# EUROPÄISCHE PATENTSCHRIFT

(11) **EP 0 697 198 B1**
(45) Veröffentlichungstag und Bekanntmachung des Hinweises auf die Patenterteilung: **15.05.2002**
(21) Anmeldenummer: 95109405.1
(22) Anmeldetag: 17.06.1995
(51) Int. Cl.: A61B 17/128

(54) **Chirurgisches Anlegegerät für Clipse**
Surgical clip applier
Applicateur de pinces chirurgicales

(30) Priorität: 17.08.1994 DE 4429084
(43) Veröffentlichungstag der Anmeldung: 21.02.1996
(73) Patentinhaber: Aesculap AG & Co. KG, 78532 Tuttlingen (DE)
(72) Erfinder: Kienzle, Karl-Ernst, 78194 Immendingen (DE); Mayenberger, RupertDipl.-Ing., D-78239 Rielasingen (DE); Nesper. Markus Dipl. Ing., 78532 Tuttlingen (DE); Weisshaupt, Dieter Dipl Ing., 78194 Immendingen (DE)
(74) Vertreter: Hoeger, Stellrecht & Partner

(56) Entgegenhaltungen:
- EP-A- 0 406 724
- EP-A- 0 409 569
- EP-A- 0 680 729
- DE-A- 4 303 544
- US-A- 4 527 726
- US-A- 5 112 343

## Beschreibung

Die Erfindung betrifft ein chirurgisches Anlegegerät für U-förmige Clipse mit einem Griff, einem daran anschließenden Rohrschaft, mit einem zangenförmigen Anlegewerkzeug am freien Ende des Rohrschafts und einem Clips-Magazin im Rohrschaft, mit einem vom Griff aus betätigbaren, im Rohrschaft angeordneten Schließmechanismus für das Anlegewerkzeug und mit einem ebenfalls vom Griff aus betätigbaren, im Rohrschaft angeordneten Vorschubmechanismus für die Clipse.

Derartige chirurgische Anlegegeräte sind besonders geeignet für die Verwendung in der Laparoskopie und in der Endoskopie, da durch den langen dünnen Rohrschaft, der beispielsweise einen Außendurchmesser von 10 mm haben kann, durch kleine Öffnungen im Körper in das Körperinnere vorgedrungen werden kann, um dort Clipse anzulegen, beispielsweise hämostatische Clipse zum Verschließen von Blutgefäßen.

Bei bekannten Clips-Anlegegeräten dieser Art, wie sie beispielsweise in der US-Patentschrift 5,084,057 oder in der US-Patentschrift 5,100,420 beschrieben sind, sind die Instrumente entweder insgesamt als Wegwerfgeräte konzipiert oder der gesamte Rohrschaft kann vom Griff abgetrennt und nach dem Anlegen der Clipse verworfen werden.

Dies ist außerordentlich aufwendig, da ganz wesentliche Teile des Clips-Anlegegeräts als Einmalgeräte ausgebildet werden müssen. Dadurch wird nicht nur der Herstellungsaufwand ganz erheblich erhöht, sondern auch der Entsorgungsaufwand.

Es ist Aufgabe der Erfindung, ein chirurgisches Anlegegerät der gattungsgemäßen Art so auszubilden, daß eine Wiederverwendung aller wesentlichen Teile möglich ist.

Diese Aufgabe wird bei einem chirurgischen Anlegegerät der eingangs beschriebenen Art erfindungsgemäß dadurch gelöst, daß der Rohrschaft eine seitliche Durchbrechung aufweist, in die das Clips-Magazin von außen lösbar derart seitlich einsetzbar ist, daß es mit dem Vorschubmechanismus in Wirkverbindung tritt und daß der Auslaß des Clips-Magazins mit einer die Clipse in das Anlegewerkzeug führenden Vorschubbahn ausgerichtet ist.

Dadurch, daß das Clips-Magazin als separates Teil in den Rohrschaft seitlich einsetzbar ist, genügt es zum Nachfüllen neuer Clipse,dieses Magazin auszuwechseln. Der Rohrschaft selber und alle in ihm angeordneten Mechanismen können wiederverwendet werden, gegebenenfalls nach einer Sterilisation.

Bei einer bevorzugten Ausführungsform ist vorgesehen, daß das Magazin im eingesetzten Zustand den Rohrschaft im Bereich der Durchbrechung vollständig ergänzt. Das Magazin bildet also praktisch den Teil des Rohrschafts aus, der in Form einer Unterbrechung am Rohrschaft fehlt, diese die Durchbrechung ausfüllende Rohrschaftwandung nimmt die Nachfüllclipse auf. Dabei ist es vorteilhaft, wenn sich die Durchbrechung über einen Umfangsbereich des Rohrschafts von bis zu 180° erstreckt. Bei einer solchen Ausgestaltung ist der Rohrschaft also über die Länge des Clips-Anlegemagazins nur halbseitig ausgebildet, die andere Hälfte des Rohrschafts wird durch das seitlich einsetzbare Clips-Magazin gebildet.

Es ist günstig, wenn sich die Durchbrechung unmittelbar an das Anlegewerkzeug anschließt und wenn sie sich insbesondere nur über einen kleineren Teil der Rohrschaftlänge erstreckt. Der übrige Aufbau des chirurgischen Anlegegeräts bleibt also im weiteren Rohrschaft vollständig unbeeinflußt von dem Clips-Magazin, dieses wird als Zubehörteil im vorderen Bereich seitlich in den Rohrschaft eingesteckt.

Dabei ist es besonders bedienerfreundlich, wenn das Clips-Magazin entsprechend einer bevorzugten Ausführungsform der Erfindung mit dem Rohrschaft durch eine elastische Schnappverbindung lösbar verbindbar ist. Es genügt daher, das Clips-Magazin seitlich an den Rohrschaft anzusetzen und durch einen Druck elastisch einschnappen zu lassen, dann ist das Gerät arbeitsbereit.

Es kann auch vorgesehen sein, daß das Clips-Magazin an seinem dem Griff zugewandten Ende einen in den Rohrschaft eintauchenden Vorsprung trägt. Insbesondere ist günstig eine Kombination eines solchen Vorsprungs und einer elastischen Schnappverbindung. Man kann dann eine besonders sichere Fixierung des Clips-Magazins dadurch erreichen, daß es einerseits mit dem Vorsprung in den Rohrschaft eintaucht und andererseits beispielsweise über zwei seitliche Wandteile den Rohrschaft umgibt und mit diesem eine Schnappverbindung eingeht.

Bei einer bevorzugten Ausführungsform ist vorgesehen, daß der Vorschubmechanismus an den Clipsen anliegende Mitnehmer aufweist, die gemeinsam um jeweils einen Clips-Abstand verschiebbar sind. Jeder Clips wird daher bei dieser Ausführungsform durch den Vorschubmechanismus positiv um eine Stellung weitergeschoben, wenn dieser betätigt wird.

Bei anderen Ausgestaltungen wäre es auch möglich, alle Clipse aneinanderliegend durch Federkraft vorzuschieben und durch Anschläge sicherzustellen, daß bei dieser Vorschubbewegung nur jeweils ein Clips nach vorn ausgeschoben wird, wie dies bei Clips-Anlegegeräten an sich bekannt ist.

Bei der bevorzugten Ausgestaltung mit separaten Mitnehmern für jeden Clips ist es vorteilhaft, wenn alle Mitnehmer an einem gemeinsamen Träger angeordnet sind, der längs des Rohrschafts verschiebbar gelagert ist.

Besonders günstig ist es, wenn die Mitnehmer federnde Zungen sind, die beim Zurückbewegen der Mitnehmer einfedern und an den folgenden Clipsen im Clips-Magazin entlanggleiten, ohne diese zu verschieben, und die beim Vorbewegen mit ihren freien Kanten an der Rückseite eines Clipses anliegen.

Dabei ist es sehr günstig, wenn die Kanten schräg zu Clips-Ebene angeordnet sind. Dies stellt sicher, daß die freien Kanten nicht nach oben oder nach unten an den Clipsen abgleiten, sondern auch bei runden Kanten der Clipse sicher an diesen anliegen.

Dabei ist es weiterhin vorteilhaft, wenn jeder Mitnehmer zwei nebeneinander angeordnete Zungen aufweist, dadurch wird eine Verkantung der Clipse beim Vorschieben verhindert.

Bei einer besonders bevorzugten Ausführungsform ist vorgesehen, daß zwischen den beiden Zungen eine weitere federnde Zunge angeordnet ist, die sich an die Unterseite des Clipses anlegt. Es ist dadurch möglich, die Mitnehmer insgesamt mit relativ hoher Federkraft gegen die Clipse anzulegen, ein zu weites Verschwenken unter der Federkraft wird durch die mittlere Zunge verhindert.

Insbesondere bei dieser Ausgestaltung ist es dann auch möglich, daß die beiden äußeren Zungen und die mittlere Zunge Teil einer elastisch gegen die Clipse vorgespannten längeren Zunge sind. Diese längere Zunge erleichtert das Entlanggleiten der Mitnehmer beim Zurückziehen derselben, und trotzdem besteht nicht die Gefahr, daß beim Vorschieben die Mitnehmer von den Clipsen abgleiten.

Zusätzlich kann bei einer bevorzugten Ausführungsform vorgesehen sein, daß der Träger zwei nach unten weisende Vorsprünge trägt, zwischen die ein den Träger verschiebender, vom Griff aus betätigbarer Mitnehmer eingreift. Dabei ist es insbesondere günstig, wenn die Vorsprünge zwei gegensinnig angeordnete federnde Zungen sind, zwischen deren freie Kante der Mitnehmer eingreift. Man erhält auf diese Weise eine besonders einfache Herstellung eines Trägers und eines Mitnehmers, der die Vorschubbewegung längs des Rohrschafts überträgt.

Besonders vorteilhaft ist es, wenn das Clips-Magazin eine zum Inneren des Rohrschafts hin offene Aufnahmekammer aufweist, in der mehrere Clipse hintereinander längs der Aufnahmekammer verschieblich aufgenommen sind, und wenn der Träger die Aufnahmekammer zum Rohrschaft hin abdeckend an dem Clips-Magazin längsverschieblich gelagert ist. Damit ist der Träger, der einen Teil des Vorschubmechanismus bildet, in das Clips-Magazin integriert und wird mit diesem eingesetzt. Eine Kopplung ist dabei besonders einfach, wenn der Träger in der beschriebenen Weise mit nach unten vorstehenden federnden Zungen versehen ist, zwischen die ein Mitnehmer des Vorschubmechanismus eingreift. Durch das Einsetzen des Clips-Magazins kann bei dieser Ausgestaltung ein sofortiger und selbsttätiger Eingriff zwischen Vorschubmechanismus einerseits und dem Träger andererseits hergestellt werden, so daß das Gerät allein durch das Einsetzen des Clips-Magazins arbeitsbereit ist.

Dabei kann der Mitnehmer mit einem mit einer Feder in die griffnahe Endstellung verschiebbaren Betätigungselement verbunden sein, so daß der Träger zwangsläufig in die Ausgangsstellung zurückgezogen wird, in der er für das Vorschieben der Clipse vorbereitet ist.

Bei einer anderen Ausführungsform ist vorgesehen, daß der Träger durch eine Feder in eine zurückgeschobene Endstellung verschoben wird und daß am Träger ein diesen entgegen der Wirkung der Feder verschiebendes Betätigungselement angreift. Bei dieser Lösung greift die Feder also direkt am Träger und nicht an einem Betätigungselement an, auch hier ist aber dafür Sorge getragen, daß der Träger durch die Feder in die Ausgangsstellung zurückgeschoben wird, in der er für ein Vorschieben der Clipse vorbereitet ist.

Bei einer bevorzugten Ausführungsform ist zusätzlich vorgesehen, daß der Träger eine Verlängerung trägt, die in seiner griffnahen, zurückgeschobenen Stellung einen Riegel betätigt, der nur im betätigten Zustand den Vorschubmechanismus freigibt, ihn aber sonst blockiert. Damit ist sichergestellt, daß bei nicht vollständig zurückgeschobenem Träger der Vorschubmechanismus nicht betätigt werden kann. Erst wenn der Träger vollständig zurückgeschoben ist, wenn also die Mitnahmezungen des Trägers hinter den vorzuschiebenden Clipsen eingerastet sind, ist eine erneute Betätigung des Vorschubmechanismus möglich.

Dieser Riegel kann beispielsweise ein schwenkbarer Hebel sein, der im unbetätigten Zustand an einem Vorsprung des Vorschubmechanismus blockierend anliegt, beispielsweise an einer Ringschulter einer Schub- und Zugstange des Vorschubmechanismus.

Das Betätigungselement kann insbesondere eine im Rohrschaft angeordnete Schub- und Zugstange sein.

Zu deren Betätigung kann am Griff ein gelenkig mit dieser verbundener Schwenkhebel gelagert sein, so daß die Schubund Zugstange und damit der Vorschubmechanismus völlig unabhängig vom Schließmechanismus für das Anlegewerkzeug betätigt werden kann.

Bei einer bevorzugten Ausführungsform ist weiterhin vorgesehen, daß der Schub- und Zugstange ein Freilauf zugeordnet ist, der die Schub- und Zugstange jeweils in einer Richtung sperrt und in der anderen freigibt, und daß die Freigaberichtung nur in einer der jeweiligen Endpositionen der Schub- und Zugstange umkehrbar ist. Ein solcher Freilauf stellt sicher, daß sowohl die Vorschubbewegung als auch die Rückholbewegung der Schub- und Zugstange in einer Richtung über den gesamten Verschiebeweg erfolgen muß, bevor eine jeweilige Rückbewegung einsetzen kann. Beim Vorschieben der Clipse werden diese also mit Sicherheit immer um einen Schritt vollständig vorgeschoben, beim Rückholen der Mitnehmer ist gewährleistet, daß sich die Mitnehmer in jedem Falle wieder hinter den nächsten Clipsen befinden, bevor die nächste Vorschubbewegung einsetzen kann.

Ein solcher Freilauf kann bei einer bevorzugten Ausführungsform gebildet werden durch einen sich schräg an eine Profilierung der Umfangsfläche der Schub- und Zugstange anlegenden, am Griff schwenkbar gelagerten Sperrhebel, der in Vertiefungen der Schub- und Zugstange am Ende der Profilierung senkrecht zur Umfangsfläche weisend eintaucht und in dieser Position beim umgekehrten Verschieben der Schub- und Zugstange von dieser in eine umgekehrt gerichtete symmetrische Schräglage verschwenkt wird. Man erhält damit auf mechanisch besonders einfache Weise einen Freilauf mit umkehrbarer Sperr-Richtung, der gleichzeitig gewährleistet, daß die Umschaltung immer nur am Ende eines vollständigen Hubs erfolgen kann.

Bei einer bevorzugten Ausführungsform der Erfindung ist vorgesehen, daß der Rohrschaft an dem der Durchbrechung gegenüberliegenden Teil abnehmbar ist. Dies erleichtert den Zugang zu den mechanischen Teilen im Inneren des Rohrschafts und erleichtert im übrigen die Reinigung und Sterilisation des Geräts.

Günstig ist es dabei, wenn der abnehmbare vordere Teil des Rohrschafts mit dessen übrigem Teil über eine Spannzangenverbindung verbunden ist.

Zusätzlich ist es vorteilhaft, wenn der Rohrschaft mit dem Griff um die Rohrschaftlängsachse drehbar und lösbar verbunden ist. Die Drehung ermöglicht eine Anlage der Clipse in unterschiedlicher Richtung, die Lösbarkeit ist ebenfalls vorteilhaft, um die Reinigung vorzunehmen oder um gegebenenfalls den Griff auch mit anders gearteten Rohrschaftinstrumenten einsetzen zu können.

Insbesondere kann die Verbindung Rohrschaft und Griff über ein Kugelgesperre erfolgen.

Günstig ist es, wenn der Schließmechanismus des Anlegewerkzeug mit zwei schwenkbar gelagerten Zangenbacken verbundene Getriebemittel aufweist, die durch eine im Rohrschaft angeordnete Schub- und Zugstange angetrieben werden.

Eine besonders platzsparende Ausgestaltung ergibt sich dabei, wenn die Schub- und Zugstange des Schließmechanismus als die Schub- und Zugstange des Vorschubmechanismus koaxial umgebende Hülse ausgebildet ist.

Zur Verschiebung dieser Hülse können am Griff zwei gegeneinander verschwenkbare Branchen angeordnet sein.

Bei einer besonders bevorzugten Ausführungsform ist vorgesehen, daß die Hülse im Bereich des Durchbruchs ausgeschnitten ist. Dadurch ist eine Verbindung zwischen der im Inneren der Hülse geführten Schub- und Zugstange des Vorschubmechanismus einerseits und dem Vorschubmechanismus selber andererseits möglich.

Die nachfolgende Beschreibung bevorzugter Ausführungsformen der Erfindung dient im Zusammenhang mit der Zeichnung der näheren Erläuterung. Es zeigen:

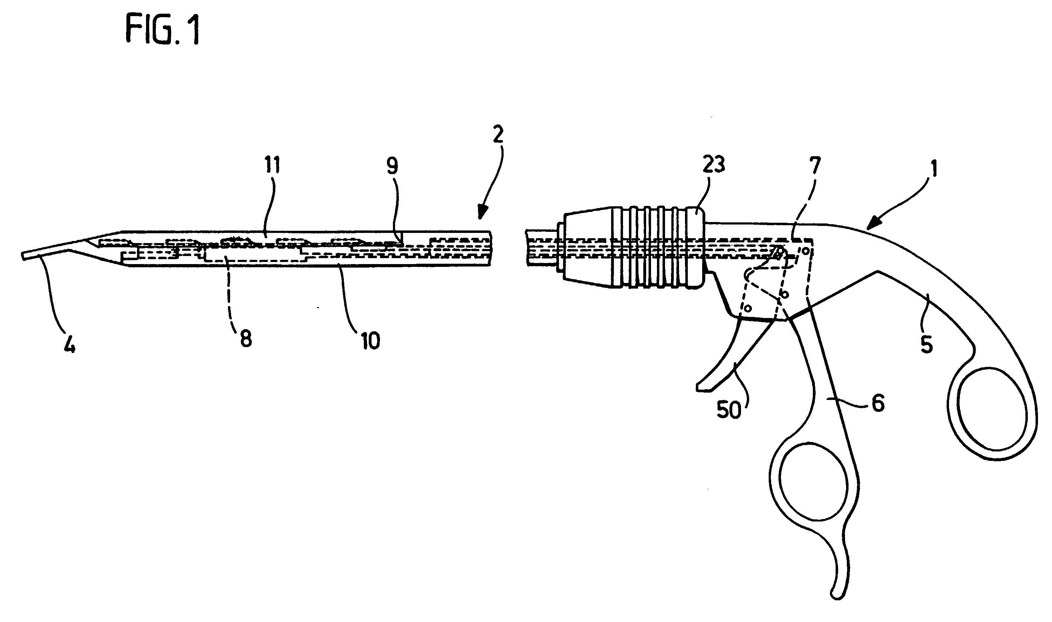

- Figur 1:: eine Seitenansicht eines Anlegegeräts für chirurgische Clipse;

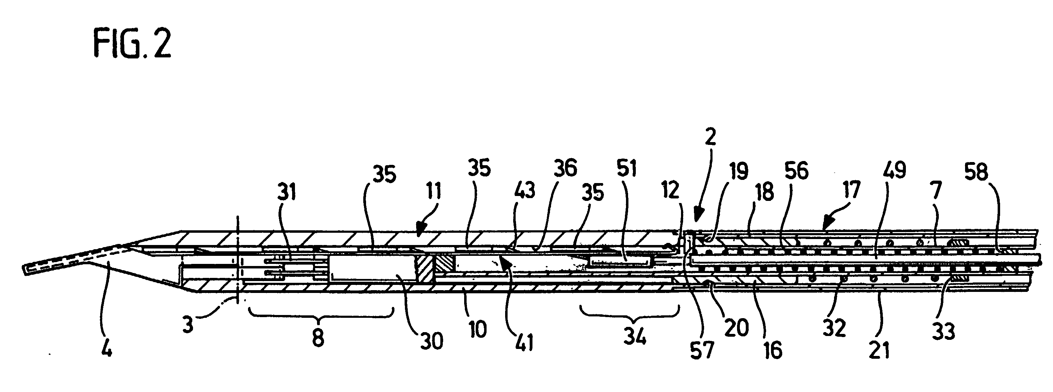

- Figur 2:: eine Längsschnittansicht des vorderen Rohrschaftteils des Anlegegeräts der Figur 1;

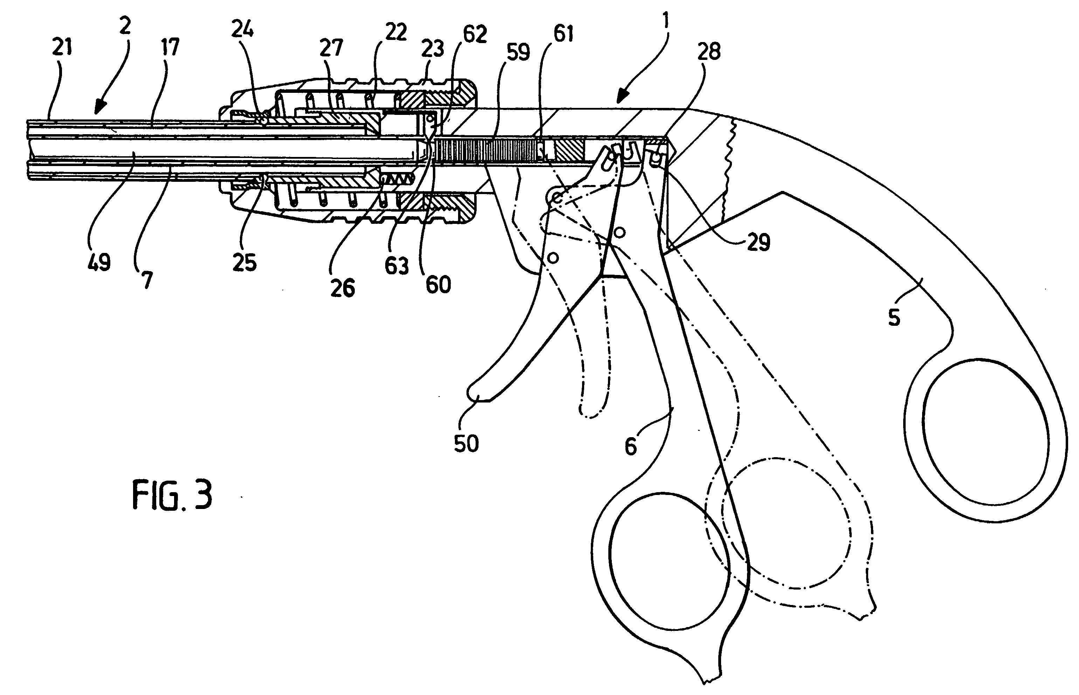

- Figur 3:: eine Längsschnittansicht des Griffs des Anlegegeräts der Figur 1;
- Figur 4:: eine nochmals vergrößerte Längsschnittansicht des vorderen Teils des Rohrschafts des Anlegegeräts der Figur 1 mit dem Vorschubmechanismus in zurückgezogener Stellung;
- Figur 5:: eine Ansicht ähnlich Figur 4 mit dem Vorschubmechanismus in vorgeschobener Stellung;
- Figur 6:: eine Ansicht ähnlich Figur 4 bei einem anderen bevorzugten Ausführungsbeispiel eines chirurgischen Anlegegeräts mit dem Vorschubmechanismus in zurückgezogener Stellung;
- Figur 7:: eine Ansicht ähnlich Figur 6 mit dem Vorschubmechanismus in vorgeschobener Stellung;
- Figur 8:: eine Schnittansicht längs Linie 8-8 in Figur 4;

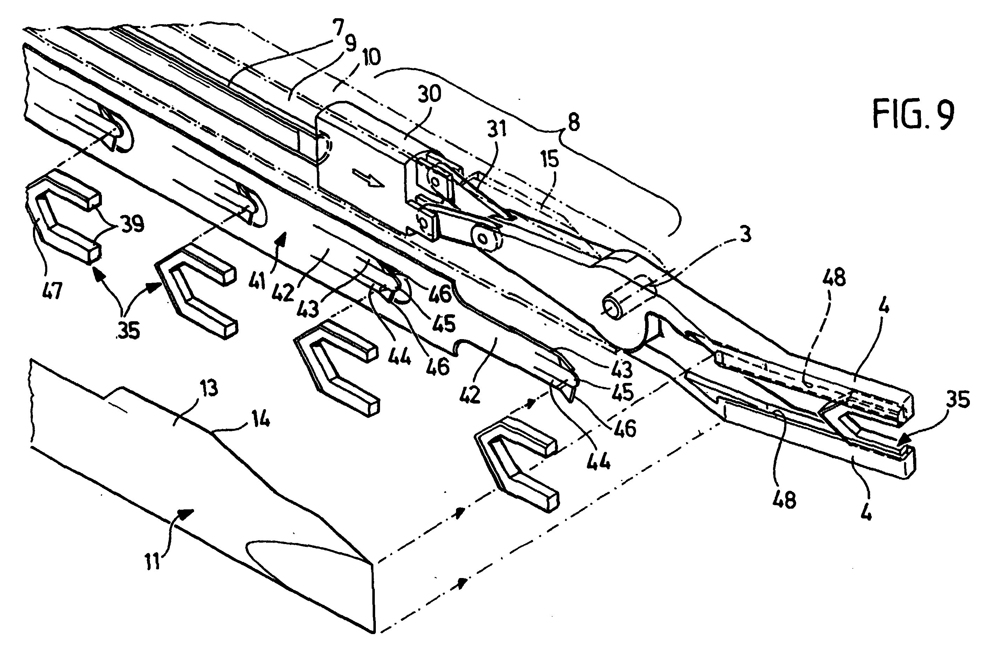

- Figur 9:: eine perspektivische Ansicht des vordersten Teils des Rohrschafts in einer Explosionsdarstellung;
- Figur 10:: eine Draufsicht auf den Unterteil des Rohrschafts im Bereich des zangenförmigen Anlegewerkzeugs und des zugehörigen Schließmechanismus mit geöffneten Schließwerkzeugen und
- Figur 11:: eine Ansicht ähnlich Figur 10 mit geschlossenen Schließwerkzeugen.

Das in der Zeichnung dargestellte Anlegegerät umfaßt einen Griff 1 und einen um seine Längsachse verdrehbar und lösbar an diesem gehaltenen Rohrschaft 2, dessen Außendurchmesser gegenüber seine Länge gering ist, beispielsweise kann die Länge 25 cm betragen, der Außendurchmesser 10 mm.

Am freien Ende des Rohrschafts 2 sind um eine gemeinsame Schwenkachse 3 verschwenkbar zwei Backen 4 eines zangenförmigen Anlegewerkzeugs gelagert, die über einen im Inneren des Rohrschafts angeordneten Schließmechanismus vom Griff 1 aus geöffnet und geschlossen werden können. Zu diesem Zweck ist der Griff 1 starr mit einer ersten Branche 5 verbunden, gegenüber welcher eine zweite Branche 6 verschwenkbar am Griff 1 gelagert ist. Diese zweite Branche 6 ist gelenkig mit einer Schub- und Zugstange in Form einer Hülse 7 verbunden, die sich durch den gesamten Rohrschaft 2 bis zu dem Schließmechanismus 8 erstreckt. Durch Verschwenken der Branchen 5 und 6 gegeneinander können also die Backen 4 des Anlegewerkzeugs geöffnet und geschlossen werden, wie dies bei Rohrschaftinstrumenten bekannt ist.

Unmittelbar anschließend an das durch die Backen 4 gebildete Anlegewerkzeug weist der Rohrschaft 2 eine sich über den vorderen Teil des Rohrschafts erstreckende Durchbrechung 9 auf, die sich in Umfangsrichtung über etwa 180° erstreckt, so daß der Rohrschaft 2 im Bereich dieser Durchbrechung 9 nur in der unteren Hälfte des Rohrschafts durch eine untere Schale 10 abgeschlossen ist, auf der Oberseite jedoch offen.

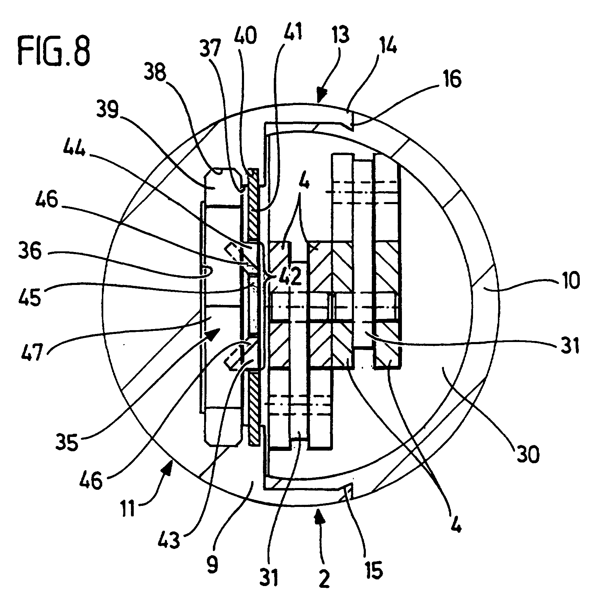

Die Durchbrechung 9 auf der Oberseite des Rohrschafts 2 kann geschlossen werden durch ein in diese Durchbrechung 9 eingesetztes Clips-Magazin 11, welches im wesentlichen einen fast halbkreisförmigen Querschnitt aufweist und welches den Querschnitt des Rohrschafts 2 zu einem Vollkreis ergänzt (Figur 8). Dieses Clips-Magazin 11 weist an seiner griffnahen Seite einen Vorsprung 12 auf, der unmittelbar angrenzend an die Durchbrechung 9 geringfügig in den Rohrschaft 2 eintaucht und dadurch die die Durchbrechung 9 begrenzende Kante untergreift. Am vorderen Teil sind seitlich an das Clips-Magazin 11 zwei Wände oder Lappen 13 angeformt, die in komplementäre Ausnehmungen der unteren Schale 10 eingreifen und im zusammengesetzten Zustand mit nach innen vorspringenden Endbereichen 14 in entsprechende Nuten 15 der unteren Schale 10 einschnappen, das heißt in diesem Bereich ergibt sich eine elastische Verrastung der Lappen 13 mit den Seitenwänden der unteren Schale 10. Durch das Einstecken des Vorsprungs 12 in den Rohrschaft 2 und durch diese Rastverbindung wird das Clips-Magazin 11 stabil am Rohrschaft 2 festgelegt, diese Verbindung ist jedoch wieder lösbar, da die Schnappverbindung durch kräftiges Abziehen des Clips-Magazins von der unteren Schale 10 wieder gelöst werden kann.

Die untere Schale 10 des Rohrschafts 2 taucht ihrerseits mit einem ringförmigen Vorsprung 16 in ein Rohr 17 ein, welches mit radial federnden Wandbereichen 18, die durch Längsschlitze voneinander getrennt sind, eine Spannzangenverbindung ausbildet. Diese Wandbereiche 18 tauchen mit ihren nach innen gebogenen Kanten 19 in eine Umfangsnut 20 des Vorsprungs 16 ein und werden in dieser Lage durch eine auf dem Rohr 17 längsverschieblich gelagerte Hülse 21 fixiert, die die Wandbereiche 18 im Bereich der in die Umfangsnut 20 eintauchenden Kanten 19 überdeckt. Die Hülse 21 kann in Längsrichtung zurückgeschoben werden, so daß die elastischen Wandbereiche 18 freigegeben werden, dann können die Kanten 19 aus der Umfangsnut 20 in radialer Richtung ausweichen, so daß der Vorsprung 16 aus dem Rohr 17 herausgezogen werden kann. Der Vorsprung 16 bildet also mit dem Rohr 17 eine Spannzangenverbindung aus, die allein durch die auf dem Rohr 17 verschiebbare Hülse 21 geschlossen und geöffnet werden kann.

Damit wird der Rohrschaft 2 durch drei Teile ausgebildet, nämlich einmal durch die untere Schale 10 und das an dieser festlegbare Clips-Magazin 11, die zusammen eine Spitze des Rohrschafts 2 bilden, und durch das Rohr 17 mit der darauf verschiebbaren Hülse 21, die den längeren Verbindungsteil zwischen der Spitze und dem Griff 1 ausbilden.

Dieses Verbindungsteil taucht in eine am Griff 1 in Längsrichtung gegen die Wirkung einer Feder 22 verschiebbare Griffhülse 23 ein, die Teil eines den Rohrschaft 2 mit dem Griff 1 verbindenden Kugelgesperres ist. Diese auf dem Griff 1 in Längsrichtung verschieblich gelagerte Griffhülse 23 legt mehrere im Griff in Radialöffnungen verschiebbare kugelförmige Verriegelungskörper 24 in einer radial innenliegenden Stellung fest, wenn die Feder 22 entspannt ist, sie ermöglicht jedoch eine radiale Auswärtsbewegung dieses Verriegelungskörpers 24, wenn die Griffhülse 23 gegen die Wirkung der Feder 22 zurückgezogen wird. In der radial eingeschobenen Stellung tauchen die Verriegelungskörper 24 in radiale Ausnehmungen 25 des Rohrs 17 ein und legen dieses in axialer Richtung am Griff fest, in ihrer radial nach außen verschobenen Stellung gegeben die Verriegelungskörper 24 aber das Rohr 17 frei, so daß es in axialer Richtung aus dem Griff 1 herausgezogen werden kann. Es ist dann auch möglich, die Hülse 21 relativ zum Rohr 17 zu verschieben, die bei im Griff 1 fixiertem Rohr 17 selber an einem Anschlag anliegt und nicht relativ zum Rohr 17 in eine Position verschoben werden könnte, in der die Spannzangenverbindung zwischen Rohr 17 und Vorsprung 16 freigegeben würde.

Das Kugelgesperre ermöglicht im übrigen eine Längsdrehung des Rohrs 17 relativ zum Griff 1, die Winkelstellung des Rohrs kann dabei in verschiedenen Zwischenstellungen fixiert werden durch eine federbeaufschlagte Kugelraste 26, die in aus der Zeichnung nicht ersichtliche Vertiefungen eines Teils 27 des Kugelgesperres eintritt.

Die im Rohrschaft 2 angeordnete Hülse 7 tritt durch die Verbindungsstelle zwischen Rohrschaft 2 und Griff 1 hindurch bis in den Griff ein und ist dort über zwei seitliche Zapfen 28, die in seitlich offene Öffnungen 29 eingreifen, gelenkig mit der Branche 6 verbunden. Beim Verschwenken der Branche 6 gegenüber der Branche 5 wird die Hülse 7 dadurch vor und zurück geschoben.

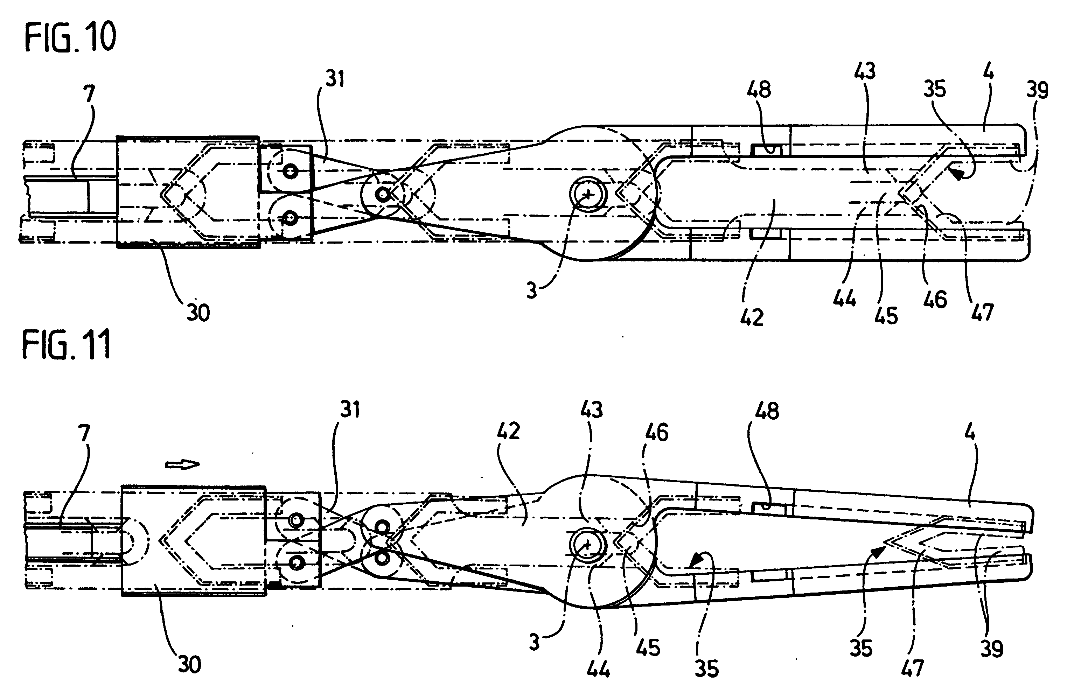

Sie ist im Bereich der Durchbrechung 9 des Rohrschafts 2 nach oben hin aufgeschnitten, so daß sie in diesem Bereich nur eine untere Schale ausbildet, die an ihrem freien Ende fest mit einem Gleitstein 30 verbunden ist, der den Querschnitt der unteren Schale 10 ausfüllend in dieser längsverschieblich gelagert ist. Dieser Gleitstein 30 ist über jeweils einen Verbindungshebel 31 gelenkig mit einer Backe 4 des Anlegewerkzeugs verbunden, so daß jeweils eine Backe 4 und ein Verbindungshebel 31 einen Kniehebel ausbilden (Figur 10). Dieser Kniehebel kann durch Vor- und Zurückschieben des Gleitsteins 30 mehr oder weniger stark eingeknickt werden, wobei eine Verschwenkung der Backen 4 eintritt. Die Anordnung und die Dimensionierung werden nun so gewählt, daß der Kniehebel beispielsweise beim Vorschieben des Gleitsteins stärker einknickt, und zwar vorzugsweise so weit, daß er bei vollständig geschlossenen Backen 4 zwischen dem Verbindungshebel 31 und der Backe 4 fast einen rechten Winkel ausbildet. Dies führt dazu, daß ein maximales Drehmoment auf die Backen 4 ausgeübt werden kann, so daß eine starke Schließkraft erzeugt wird. Beim Zurückziehen des Gleitsteins 30 erfolgt wieder eine Öffnung der Backen 4. Diese Öffnung wird im übrigen unterstützt durch eine Schraubenfeder 32, die die Hülse 7 koaxial umgibt und sich mit einem Ende an dem in das Rohr 17 eintauchenden Vorsprung 16 und am anderen Ende an einer Ringschulter 33 abstützt, die dauerhaft mit der Hülse 7 verbunden ist. Dadurch wird die Hülse 7 in Richtung auf den Griff 1 mit einer Federkraft beaufschlagt, die die Öffnung der Backen 4 unterstützt.

Der den Gleitstein 30, die Verbindungshebel 31 und den rückwärtigen Teil der Backen 4 umfassende Schließmechanismus 8 ist vollständig in der unteren Schale 10 untergebracht und läßt somit die gesamte Durchbrechung 9 frei zur Aufnahme eines Vorschubmechanismus 34 für die Clipse 35 und zur Aufnahme des Clips-Magazins 11, in dem sich die Clipse 35 befinden.

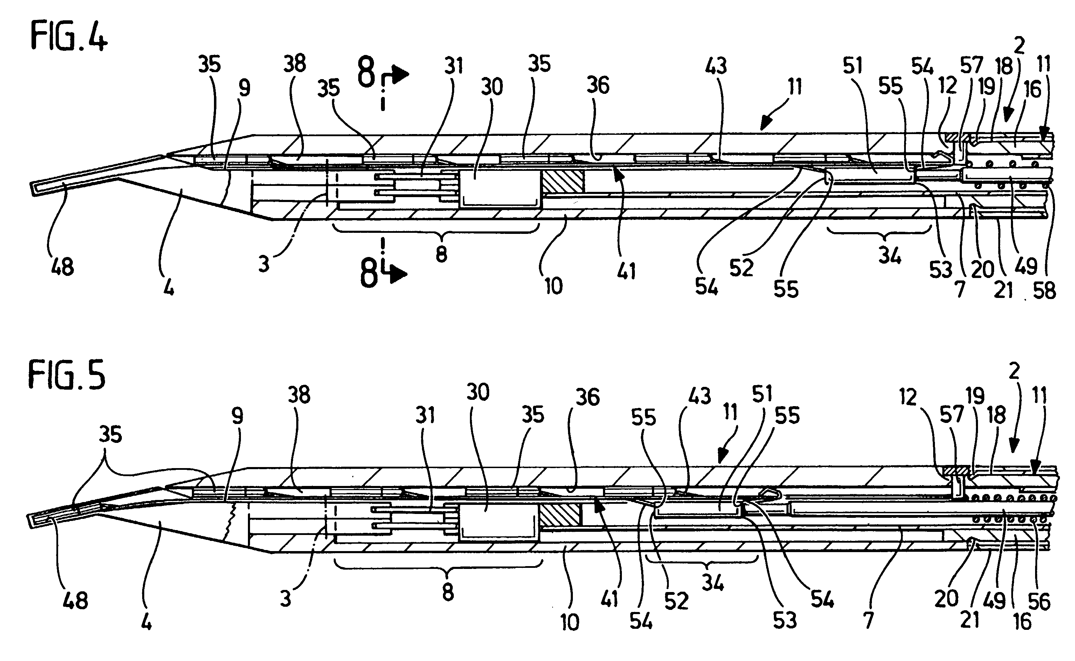

In dem Clips-Magazin 11 befindet sich eine nach unten hin offene Aufnahmekammer 36 für die Clipse 35, die die Form einer Längsnut im Clips-Magazin 11 aufweist. In die Seitenwände dieser Aufnahmekammer 36 sind einander gegenüberliegend Führungsnuten 38 eingelassen, in welche die Schenkel 39 der U-förmigen Clipse 35 eintauchen. Mehrere dieser U-förmigen Clipse 35 sind auf diese Weise hintereinander in der Aufnahmekammer 36 angeordnet, wobei sie relativ zueinander einen Abstand einhalten (Figur 4).

Parallel zu den Führungsnuten 38 verläuft in den Seitenwänden 37 jeweils eine weitere Führungsnut 40, und in diesen ist längsverschieblich ein sich über die gesamte Länge des Clips-Magazins 11 erstreckender, flächiger oder plattenförmiger Träger 41 angeordnet, der die Aufnahmekammer 36 nach unten hin abschließt.

Der Träger 41 weist in Längsrichtung mehrere ausgestanzte Federzungen 42 auf, die jeweils an ihrem vorderen Ende durch Längsschnitte in drei nebeneinander angeordnete Federzungen 43, 44 und 45 aufgeteilt sind. Die beiden äußeren Federzungen 43 und 44 sind im Bereich ihres freien Endes zur Aufnahmekammer 36 hin schräg abgebogen, so daß die freien Kanten 46 zur Fläche des Trägers 41 schräg verlaufen und über diese Fläche nach oben zur Aufnahmekammer 36 hin vorstehen.

Wenn der Träger 41 sich in seiner zurückgezogenen Position befindet, liegen diese Kanten 46 jeweils an der die beiden Schenkel 39 verbindenden Brücke 47 eines Clipses 35 an, wobei die schräg verlaufenden Kanten 46 sicherstellen, daß auch bei einer linienförmigen Kontur der Brücke 47 eine sichere Anlage erfolgt und ein Abgleiten der Kanten 46 vermieden wird. Die mittlere Federzunge 45 der Federzunge 42 legt sich an die Unterseite der Clipse an, so daß auf diese Weise auch sicher vermieden wird, daß die Kanten an der Oberseite über die Clipse hinweggleiten.

Wenn der Träger 41 nach vorne vorgeschoben wird, nimmt somit jedes Paar von Federzungen 43, 44 einen Clips 35 mit und schiebt diesen im Clips-Magazin 11 um die Schrittweite des Verschiebung des Trägers 41 nach vorne.

Der vorderste Clips 35 wird dabei aus den Führungsnuten 38 in den Seitenwänden 37 der Aufnahmekammer 36 nach vorne ausgeschoben und gelangt in mit den Führungsnuten 38 ausgerichtete, sich daran anschließende Führungsnuten 48 an den Innenseiten der Backen 4.

An seinem vorderen Ende läuft der Träger 41 in einer Federzunge 42 aus, die insgesamt schmaler ausgebildet ist als der Träger 41. Beim Vorschieben des Trägers 41 tritt diese Federzunge 42 zwischen die Backen 4 ein und schiebt den Clips 35 in die etwas nach unten geneigten Nuten 48 der Backen 4 bis an deren vorderes Ende. Durch die schmale federnde Ausführung der Federzunge 42 im vorderen Teils kann diese ohne weiteres der etwas geänderten Neigung der Führungsnuten 48 folgen.

In dieser vordersten Position verbleibt der vorderste Clips 35, wenn der Träger 41 seine maximale Vorschubbewegung beendet hat. Wird der Träger 41 dann wieder zurückgezogen, so gleiten die Federzungen 42 an den nachfolgenden Clipsen 35 entlang, bis sie wieder vollständig hinter diesen stehen und wieder in die Mitnahmeposition nach oben federn können, in denen die Kante 46 an den Brücken 47 der Clipse 35 anliegen. In den Teilen des Clips-Magazins 11, in denen sich kein Clips 35 befindet, gleiten die Federzungen 43 und 44 gegebenenfalls am Boden der Aufnahmekammer 36 entlang ohne einen Clips mitzunehmen, die Bewegung des Trägers 41 wird dadurch in keiner Weise behindert.

Es wird damit deutlich, daß allein durch eine Vor- und Rückbewegung des Träger 41 sukzessive jeweils ein Clips 35 zwischen die Backen 4 eingeschoben werden kann, der dann in der beschriebene Weise durch Schließen der Backen angelegt werden kann, beispielsweise zum Verschließen eines Blutgefäßes.

Dies erfolgt, bis das Clips-Magazin 11 geleert ist, anschließend kann in einfacher Weise ein neues Clips-Magazin 11 anstelle des geleerten eingesetzt und die Tätigkeit fortgeführt werden.

Die Verschiebung des Trägers 41 erfolgt durch den Vorschubmechanismus 34, und dieser kann sehr einfach aufgebaut sein. Er umfaßt bei einem ersten Ausführungsbeispiel gemäß der Darstellung der Figuren 4 und 5 eine im Inneren der Hülse 7 längsverschieblich geführte Schub- und Zugstange 49, die gelenkig mit einem Schwenkhebel 50 am Griff 1 verbunden ist. Dessen Schwenkbewegung führt dazu, daß die Schub- und Zugstange 49 im Inneren des Rohrschafts und im Inneren der Hülse 7 verschoben werden kann. Die Schubund Zugstange 49 trägt an ihrem freien Ende einen Mitnehmer 51 mit einer vorderen und einer hinteren, senkrecht zur Verschieberichtung verlaufenden Anlagefläche 52 beziehungsweise 53, an denen vom Träger 41 in Gegenrichtung verlaufende, nach unten vorstehende Federzungen 54 mit ihren Kanten 55 anliegen.

Durch die Verwendung dieser Federzungen 54 ist es möglich, den Mitnehmer 51 nach dem Einsetzen eines Clips-Magazins 11 einfach dadurch in Eingriff mit dem Träger zu bringen, daß der Mitnehmer in Längsrichtung verschoben wird, er gleitet dann auf den Federzungen 54 auf, bis er die Mitnahmeposition erreicht hat, und in dieser halten ihn die Kanten 55 der Federzungen 54 in beiden Richtungen.

Die Schub- und Zugstange 49 wird von einer Schraubenfeder 56 konzentrisch umgeben, die sich einerseits an einem Vorsprung 57 des Rohrschafts 2 und andererseits an einer Stufe 58 der Schub- und Zugstange 49 abstützt und diese dadurch in die zurückgeschobene Stellung verschiebt. Ein Vorschieben des Trägers 41 in Richtung auf das freie Ende des Rohrschafts 2 erfolgt somit gegen die Wirkung der Schraubenfeder 56, die sich im übrigen im Inneren der Hülse 7 befindet.

Im Bereich des Griffs 1 trägt die Schub- und Zugstange 49 über eine Länge, die im wesentlichen ihrem Hub entspricht, eine Profilierung 59 im Form von Umfangsrillen, an die sich an beiden Enden jeweils eine Umfangsnut 60, 61 anschließt.

Im Griff 1 ist ein im wesentlichen senkrecht zur Rohrschaftlängsachse angeordneter Sperr-Riegel 62 verschwenkbar gelagert, der mit seiner Spitze 63 geringfügig in die Umfangsnuten 60 und 61 eintaucht, wenn diese ihm genau gegenüberstehen. Wird die Schub- und Zugstange 49 ausgehend von einer solchen Position verschoben, verschwenkt der Bereich der Profilierung 59 den Sperr-Riegel 62 in leichte Schräglage, bei welcher die Spitze 63 in Richtung der Verschiebung weist. Diese Spitze 63 gleitet dabei auf der Profilierung 59 entlang und verhindert dabei eine Bewegung der Schub- und Zugstange 49 in der umgekehrten Richtung, da dabei die Spitze 63 in die Umfangsnuten der Profilierung 59 eintritt. Die Schub- und Zugstange 49 muß daher in der ursprünglichen Richtung weitergeschoben werden, bis die Spitze 63 des Sperr-Riegels 62 in die jeweilige andere Umfangsnut eintaucht und dann eine umgekehrte Verschiebung zuläßt. Man erhält auf diese Weise mit einfachsten mechanischen Mitteln einen Freilauf der Schub- und Zugstange 49, der eine Verschiebung jeweils nur in eine Richtung erlaubt und dessen Verschieberichtung immer dann umgeschaltet wird, wenn die Schub- und Zugstange ihren Hub in eine Richtung vollständig vollendet hat.

Damit wird sichergestellt, daß die Schieberichtung des Trägers 41 erst umgekehrt werden kann, wenn die vorhergehende Verschiebung vollständig durchgeführt worden ist, wenn also entweder ein Clips 35 vollständig in die Anlageposition zwischen den Backen 4 eingeschoben worden ist oder wenn die Federzungen 43 und 44 hinter einen Clips vollständig zurückgezogen worden sind.

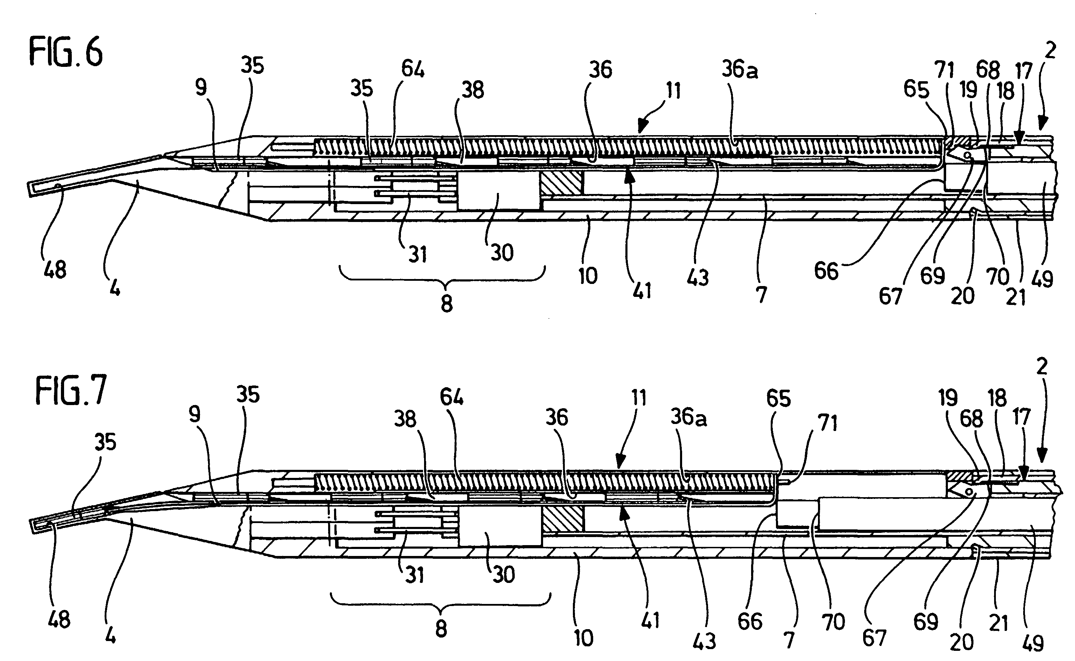

Bei dem Ausführungsbeispiel der Figuren 6 und 7 ist ein ähnlicher Aufbau des Clips-Magazins gewählt, einander entsprechende Teile tragen daher dieselben Bezugszeichen. Im Unterschied zum Ausführungsbeispiel der Figuren 4 und 5 ist im Clips-Magazin 11 zusätzlich in einer oberhalb der Aufnahmekammer 36 angeordneten Öffnung 36a eine Schraubenfeder 64 eingelegt, die sich einseitig an dem Clips-Magazin 11 und mit dem anderen Ende an einem nach oben abgebogenen Ende 65 des Träger 41 abstützt. Dadurch wird der Träger 41 bereits im Clips-Magazin 11 in die zurückgezogene Position verschoben.

Die Schub- und Zugstange 49 greift an dem Träger 41 nicht über einen Mitnehmer 51 und Federzungen 54 an, sondern liegt lediglich mit ihrer Stirnfläche 66 am Träger 41 an. Durch das Vorsehen einer Schraubenfeder 64 bereits im Clips-Magazin 11 genügt es, wenn die Schub- und Zugstange 49 den Träger nach vorne verschiebt, die Rückbewegung wird allein von der Schraubenfeder 64 vorgenommen. Es ist daher auch kein in beiden Richtungen formschlüssiger Eingriff der Schub- und Zugstange 49 und des Trägers 41 notwendig.

Bei dem Ausführungsbeispiel der Figuren 6 und 7 sind außerdem zusätzliche Sicherungsmittel vorgesehen, die ein Vorschieben der Schub- und Zugstange 49 verhindern, wenn der Träger 41 nicht in der vollständig zurückgezogenen Stellung steht. Dazu ist am Rohrschaft 2 im Bereich der hinteren Kante der Durchbrechung 9 ein Kipphebel 67 schwenkbar gelagert, der durch eine Blattfeder 68 mit seiner rückwärtigen unteren Kante 69 gegen die Schub- und Zugstange 49 gedrückt wird. Diese weist im Bereich ihres vorderen Endes eine Stufe 70 auf, an der sich die Kante 69 anlegen kann, so daß dadurch ein Vorschieben der Schubund Zugstange 49 verhindert wird.

Der Kipphebel 67 kann durch eine Verlängerung 71 am Träger 41 gegen die Wirkung der Blattfeder 68 so weit verschwenkt werden, daß die Kante 69 die Stufe 70 freigibt und daß eine freie Verschiebung der Schub- und Zugstange 49 möglich ist. Selbstverständlich ist eine solche Freigabe nur möglich, wenn der Träger 41 in der vollständig zurückgeschobenen Stellung steht, andernfalls bleibt der Kipphebel 67 in der Blockierstellung.

Damit ist sichergestellt, daß die Schub- und Zugstange 49 nicht verschoben werden kann, wenn gar kein Clips-Magazin 11 eingesetzt ist oder wenn der Träger 41 noch nicht vollständig zurückgeschoben ist.

## Patentansprüche

1. Chirurgisches Anlegegerät für U-förmige Clipse (35) mit einem Griff (1), einem daran anschließenden Rohrschaft (2), mit einem zangenförmigen Anlegewerkzeug (4) am freien Ende des Rohrschaftes (2) und einem Clips-Magazin (11) im Rohrschaft (2), mit einem vom Griff (1) aus betätigbaren, im Rohrschaft (2) angeordneten Schließmechanismus für das Anlagewerkzeug (4) und mit einem ebenfalls vom Griff (1) aus betätigbaren, im Rohrschaft (2) angeordneten Vorschubmechanismus für die Clipse (35), **dadurch gekennzeichnet, daß** der Rohrschaft (2) eine seitliche Durchbrechung (9) aufweist, in die das Clips-Magazin (11) von außen lösbar derart seitlich einsetzbar ist, daß es mit dem Vorschubmechanismus (34) in Wirkverbindung tritt und daß der Auslaß des Clips-Magazins (11) mit einer die Clipse (35) in das Anlegewerkzeug (4) führenden Vorschubbahn (48) ausgerichtet ist.

2. Chirurgisches Anlegegerät nach Anspruch 1, **dadurch gekennzeichnet, daß** das Clips-Magazin (11) im eingesetzten Zustand den Rohrschaft (2) im Bereich der Durchbrechung (9) vollständig ergänzt.

3. Chirurgisches Anlegegerät nach einem der Ansprüche 1 oder 2, **dadurch gekennzeichnet, daß** sich die Durchbrechung (9) über einen Umfangsbereich des Rohrschafts (2) von bis zu 180° erstreckt.

4. Chirurgisches Anlegegerät nach einem der voranstehenden Ansprüche, **dadurch gekennzeichnet, daß** die Durchbrechung (9) sich unmittelbar an das Anlegewerkzeug (4) anschließt.

5. Chirurgisches Anlegegerät nach einem der voranstehenden Ansprüche, **dadurch gekennzeichnet, daß** sich die Durchbrechung (9) nur über einen kleineren Teil der Rohrschaftlänge erstreckt.

6. Chirurgisches Anlegegerät nach einem der voranstehenden Ansprüche, **dadurch gekennzeichnet, daß** das Clips-Magazin (11) mit dem Rohrschaft (2) durch eine elastische Schnapp-Verbindung (13, 15, 16) lösbar verbindbar ist.

7. Chirurgisches Anlegegerät nach einem der voranstehenden Ansprüche, **dadurch gekennzeichnet, daß** das Clips-Magazin (11) an seinem dem Griff (1) zugewandten Ende einen in den Rohrschaft (2) eintauchenden Vorsprung (12) trägt.

8. Chirurgisches Anlegegerät nach einem der voranstehenden Ansprüche, **dadurch gekennzeichnet, daß** der Vorschubmechanismus (34) an den Clipsen (35) anliegende Mitnehmer (43, 44) aufweist, die gemeinsam um jeweils einen Clipsabstand verschiebbar sind.

9. Chirurgisches Anlegegerät nach Anspruch 8, **dadurch gekennzeichnet, daß** alle Mitnehmer (43, 44) an einem gemeinsamen Träger (41) angeordnet sind.

10. Chirurgisches Anlegegerät nach Anspruch 8 oder 9, **dadurch gekennzeichnet, daß** die Mitnehmer (43, 44) federnde Zungen sind, die beim Zurückbewegen der Mitnehmer einfedern und an den folgenden Clipsen (35) im Clips-Magazin (11) entlanggleiten ohne diese zu verschieben, und die beim Vorbewegen mit ihren freien Kanten (46) an der Rückseite eines Clipses (35) anliegen.

11. Chirurgisches Anlegegerät nach Anspruch 10, **dadurch gekennzeichnet, daß** die Kanten (46) schräg zur Clips-Ebene angeordnet sind.

12. Chirurgisches Anlegegerät nach Anspruch 10 oder 11, **dadurch gekennzeichnet, daß** jeder Mitnehmer zwei nebeneinander angeordnete Zungen (43, 44) aufweist.

13. Chirurgisches Anlegegerät nach Anspruch 12, **dadurch gekennzeichnet, daß** zwischen den beiden Zungen (43, 44) eine weitere federnde Zunge (45) angeordnet ist, die sich an die Unterseite des Clipses (35) anlegt.

14. Chirurgisches Anlegegerät nach Anspruch 13, **dadurch gekennzeichnet, daß** die beiden äußeren Zungen (43, 44) und die mittlere Zunge (45) Teil einer elastisch gegen die Clipse (35) vorgespannten längere Zunge (42) sind.

15. Chirurgisches Anlegegerät nach einem der Ansprüche 9 bis 14, **dadurch gekennzeichnet, daß** der Träger (41) zwei nach unten weisende Vorsprünge (54) trägt, zwischen die ein den Träger (41) verschiebender, vom Griff (1) aus betätigbarer Mitnehmer (51) eingreift.

16. Chirurgisches Anlegegerät nach Anspruch 15, **dadurch gekennzeichnet, daß** die Vorsprünge zwei gegensinnig angeordnete federnde Zungen (54) sind, zwischen deren freien Kanten (55) der Mitnehmer (51) eingreift.

17. Chirurgisches Anlegegerät nach einem der Ansprüche 9 bis 16, **dadurch gekennzeichnet, daß** das Clips-Magazin (11) eine zum Inneren des Rohrschafts (2) hin offene Aufnahmekammer (36) aufweist, in der mehrere Clipse (35) hintereinander längs der Aufnahmekammer (36) verschieblich aufgenommen sind, und daß der Träger (41) die Aufnahmekammer (36) zum Rohrschaft (2) hin abdeckend an dem Clips-Magazin (11) längsverschieblich gelagert ist.

18. Chirurgisches Anlegegerät nach einem der Ansprüche 15 bis 17, **dadurch gekennzeichnet, daß** der Mitnehmer (51) mit einem mit einer Feder (56) in die griffnahe Endstellung verschiebbaren Betätigungselement (49) verbunden ist.

19. Chirurgisches Anlegegerät nach einem der Ansprüche 9 bis 14 oder 17, **dadurch gekennzeichnet, daß** der Träger (41) durch eine Feder (64) in eine zurückgeschobene Endstellung verschoben wird und daß am Träger (41) ein diesen entgegen der Wirkung der Feder (64) verschiebendes Betätigungselement (49) angreift.

20. Chirurgisches Anlegegerät nach Anspruch 19, **dadurch gekennzeichnet, daß** der Träger (41) eine Verlängerung (71) trägt, die in seiner griffnahen, zurückgezogenen Stellung einen Riegel (67) betätigt, der nur im betätigten Zustand der Vorschubmechanismus (34) freigibt, ihn aber sonst blockiert.

21. Chirurgisches Anlegegerät nach Anspruch 20, **dadurch gekennzeichnet, daß** der Riegel (67) ein Kipphebel ist, der im unbetätigten Zustand an einem Vorsprung (57) des Vorschubmechanismus (34) blockierend anliegt.

22. Chirurgisches Anlegegerät nach einem der Ansprüche 18 bis 21, **dadurch gekennzeichnet, daß** das Betätigungselement eine im Rohrschaft (2) angeordnete Schub- und Zugstange (49) ist.

23. Chirurgisches Anlegegerät nach Anspruch 22, **dadurch gekennzeichnet, daß** am Griff (1) ein gelenkig mit der Schub- und Zugstange (49) verbundener Schwenkhebel (50) gelagert ist.

24. Chirurgisches Anlegegerät nach einem der Ansprüche 22 oder 23, **dadurch gekennzeichnet, daß** der Schubund Zugstange (49) ein Freilauf (59, 60, 61, 62) zugeordnet ist, der die Schub- und Zugstange (49) jeweils in eine Richtung sperrt und in der anderen freigibt, und daß die Freigaberichtung nur in einer der jeweiligen Endpositionen der Schub- und Zugstange (49) umkehrbar ist.

25. Chirurgisches Anlegegerät nach Anspruch 24, **dadurch gekennzeichnet, daß** der Freilauf gebildet wird durch einen sich schräg an eine Profilierung (59) der Umfangsfläche der Schub- und Zugstange (49) anlegenden, am Griff (1) schwenkbar gelagerten Sperrhebel (62), der in Vertiefungen (60, 61) der Schub- und Zugstange (49) am Ende der Profilierung (59) senkrecht zur Umfangsfläche weisend eintaucht und in dieser Position beim umgekehrten Verschieben der Schub- und Zugstange (49) von dieser in eine umgekehrt gerichtete, symmetrische Schräglage verschwenkt wird.

26. Chirurgisches Anlegegerät nach einem der voranstehenden Ansprüche, **dadurch gekennzeichnet, daß** der Rohrschaft (2) in dem der Durchbrechung (9) gegenüberliegenden Teil (10) abnehmbar ist.

27. Chirurgisches Anlegegerät nach Anspruch 26, **dadurch gekennzeichnet, daß** der abnehmbare vordere Teil (10) des Rohrschafts (2) mit dessen übrigem Teil (17, 21) über eine Spannzangenverbindung (18, 19, 20) verbunden ist.

28. Chirurgisches Anlegegerät nach einem der voranstehenden Ansprüche, **dadurch gekennzeichnet, daß** der Rohrschaft (2) mit dem Griff (1) um die Rohrschaftlängsachse drehbar und lösbar verbunden ist.

29. Chirurgisches Anlegegerät nach Anspruch 28, **dadurch gekennzeichnet, daß** die Verbindung zwischen Rohrschaft (2) und Griff (1) über ein Kugelgesperre (22, 23, 24, 25) erfolgt.

30. Chirurgisches Anlegegerät nach einem der voranstehenden Ansprüche, **dadurch gekennzeichnet, daß** der Schließmechanismus (8) des Anlegewerkzeugs (4) mit zwei schwenkbar gelagerten Zangenbacken verbundene Getriebemittel aufweist, die durch eine im Rohrschaft (2) angeordnete Schub- und Zugstange angetrieben werden.

31. Chirurgisches Anlegegerät nach Anspruch 30, **dadurch gekennzeichnet, daß** die Schub- und Zugstange des Schließmechanismus (8) als die Schub- und Zugstange (49) des Vorschubmechanismus (34) koaxial umgebende Hülse (7) ausgebildet ist.

32. Chirurgisches Anlegegerät nach Anspruch 31, **dadurch gekennzeichnet, daß** zur Verschiebung der Hülse (7) am Griff (1) zwei gegeneinander verschwenkbare Branchen (5, 6) angeordnet sind.

33. Chirurgisches Anlegegerät nach Anspruch 31 oder 32, **dadurch gekennzeichnet, daß** die Hülse (7) im Bereich des Durchbruchs (9) ausgeschnitten ist.

## Claims

1. A surgical applicator for U-shaped clips (35), comprising a handle (1), a tubular shaft (2) adjacent thereto, a forceps-type applicator implement (4) at the free end of the tubular shaft (2) and a clip magazine (11) arranged therein, a closing mechanism for the applicator implement (4) and arranged in the tubular shaft (2) and actuatable from the handle (1), and a feed mechanism for the clips (35) and likewise arranged in the tubular shaft (2) and actuatable from the handle (1), **characterised in that** the tubular shaft (2) has a lateral opening (9), into which the clip magazine (11) is removably laterally insertable from the outside in such a way that it is operatively connected to the feed mechanism (34), and **in that** the outlet of the clip magazine (11) is provided with a feed path (48) guiding the clips (35) into the applicator implement (4).

2. A surgical applicator according to claim 1, **characterised in that** the clip magazine (11) in the inserted state fully complements the tubular shaft (2) in the region of the opening (9).

3. A surgical applicator according to either one of claims 1 and 2, **characterised in that** the opening (9) extends over a circumferential region of the tubular shaft (2) of up to 180°.

4. A surgical applicator according to any one of the preceding claims, **characterised in that** the opening (9) is directly adjacent to the applicator implement (4).

5. A surgical applicator according to any one of the preceding claims, **characterised in that** the opening (9) only extends over a relatively small portion of the length of the tubular shaft.

6. A surgical applicator according to any one of the preceding claims, **characterised in that** the clip magazine (11) is detachably connectable to the tubular shaft (2) by means of a resilient snap-locking connection (13, 15, 16).

7. A surgical applicator according to any one of the preceding claims, **characterised in that** the clip magazine (11) has a projection (12) extending into the tubular shaft (2) at its end facing the handle (1).

8. A surgical applicator according to any one of the preceding claims, **characterised in that** the feed mechanism (34) has retainers (43, 44) resting against the clips (35) and jointly displaceable by a clip distance.

9. A surgical applicator according to claim 8, **characterised in that** all the retainers (43, 44) are arranged on a common carrier (41).

10. A surgical applicator according to claim 8 or 9, **characterised in that** the retainers (43, 44) are resilient tongues which spring inwards when the retainers are moved back and slide along the succeeding clips (35) in the clip magazine (11) without displacing them and which rest against the rear of a clip (35) with their free edges (46) when moved forwards.

11. A surgical applicator according to claim 10, **characterised in that** the edges (46) are arranged obliquely to the clip plane.

12. A surgical applicator according to claim 10 or 11, **characterised in that** each retainer has two tongues (43, 44) arranged side by side.

13. A surgical applicator according to claim 12, **characterised in that** a further resilient tongue (45) is arranged between the two tongues (43, 44) and rests against the underside of the clip (35).

14. A surgical applicator according to claim 13, **characterised in that** the two outer tongues (43, 44) and the middle tongue (45) are part of a longer tongue (42) resiliently pretensioned against the clips (35).

15. A surgical applicator according to any one of claims 9 to 14, **characterised in that** the carrier (41) has two downwardly extending projections (54), between which engages a retainer (51) displacing the carrier (41) and actuatable from the handle (1).

16. A surgical applicator according to claim 15, **characterised in that** the projections are two opposing resilient tongues (54), between the free edges (55) of which the retainer (51) engages.

17. A surgical applicator according to any one of claims 9 to 16, **characterised in that** the clip magazine (11) has a holding chamber (36) which is open towards the interior of the tubular shaft (2) and in which a plurality of clips (35) are displaceably held one behind the other along the holding chamber (36), and **in that** the carrier (41) is longitudinally displaceably mounted on the clip magazine (11) so as to cover the holding chamber (36) in the direction of the tubular shaft (2).

18. A surgical applicator according to any one of claims 15 to 17, **characterised in that** the retainer (51) is connected to an actuating member (49) displaceable by a spring (56) into the end position near the handle.

19. A surgical applicator according to any one of claims 9 to 14 or 17, **characterised in that** the carrier (41) is displaced by a spring (64) into a retracted end position, and **in that** an actuating member (49) displacing the carrier (41) against the action of the spring (64) engages the carrier (41).

20. A surgical applicator according to claim 19, **characterised in that** the carrier (41) carries an extension (71) which, in the retracted position of the carrier near the handle, actuates a lock (67) which releases the feed mechanism (34) only in the actuated state, but otherwise locks it.

21. A surgical applicator according to claim 20, **characterised in that** the lock (67) is a rocker arm which, in the non-actuated state, rests against the projection (57) of the feed mechanism (34) and locks it.

22. A surgical applicator according to any one of claims 18 to 21, **characterised in that** the actuating member is a push-pull rod (49) arranged in the tubular shaft (2).

23. A surgical applicator according to claim 22, **characterised in that** a pivoted lever (50), connected in an articulated manner to the push-pull rod (49), is mounted on the handle (1).

24. A surgical applicator according to either one of claims 22 and 23, **characterised in that** a freewheel mechanism (59, 60, 61, 62) is associated with the push-pull rod (49) and locks it in one direction and releases it in the other, and **in that** the release direction is reversible only in one of the respective end positions of the push-pull rod (49).

25. A surgical applicator according to claim 24, **characterised in that** the freewheel mechanism is formed by a locking lever (62) which rests obliquely against profiling (59) of the circumferential surface of the push-pull rod (49), is pivotably mounted on the handle (1), extends into recesses (60, 61) of the push-pull rod (49) at the end of the profiling (59) perpendicularly to the circumferential surface and, in this position, during the reverse displacement of the push-pull rod (49), is pivoted by the latter into a reverse symmetrical oblique position.

26. A surgical applicator according to any one of the preceding claims, **characterised in that** the part (10) of the tubular shaft (2) opposite the opening (9) is removable.

27. A surgical applicator according to claim 26, **characterised in that** the removable front part (10) of the tubular shaft (2) is connected to the remainder (17, 21) thereof by means of a chuck connection (18, 19, 20).

28. A surgical applicator according to any one of the preceding claims, **characterised in that** the tubular shaft (2) is detachably connected to the handle (1) so as to be rotatable about the longitudinal axis of the tubular shaft.

29. A surgical applicator according to claim 28, **characterised in that** the connection between the tubular shaft (2) and the handle (1) is produced by a spherical locking mechanism (22, 23, 24, 25).

30. A surgical applicator according to any one of the preceding claims, **characterised in that** the closing mechanism (8) of the applicator implement (4) has transmission means connected to two pivotably mounted forceps jaws and driven by a push-pull rod arranged in the tubular shaft (2).

31. A surgical applicator according to claim 30, **characterised in that** the push-pull rod of the closing mechanism (8) is formed as a sleeve (7) co-axially surrounding the push-pull rod (49) of the feed mechanism (34).

32. A surgical applicator according to claim 31, **characterised in that** two mutually pivotable arms (5, 6) are arranged on the handle (1) for displacement of the sleeve (7).

33. A surgical applicator according to claim 31 or 32, **characterised in that** the sleeve (7) is cut out in the region of the opening (9).

## Revendications

1. Applicateur chirurgical pour agrafes (35) en forme de U, comportant une poignée (1), une tige tubulaire (2) qui s'y raccorde, un outil applicateur en forme de pince (4) à l'extrémité libre de la tige tubulaire (2) et une cartouche d'agrafes (11) dans la tige tubulaire (2), un mécanisme de fermeture pour l'outil applicateur (4), agencé dans la tige tubulaire (2) et susceptible d'être actionné depuis la poignée (1), et un mécanisme d'avancement pour les agrafes (35), également agencé dans la tige tubulaire (2) et susceptible d'être actionné depuis la poignée (1), **caractérisé en ce que** la tige tubulaire (2) présente une percée (9) latérale dans laquelle on peut insérer latéralement depuis l'extérieur la cartouche d'agrafes (11) de manière détachable, de sorte qu'elle entre en coopération avec le mécanisme d'avancement et que la sortie de la cartouche d'agrafes (11) est alignée avec une trajectoire d'avancement (48) guidant les agrafes (35) dans l'outil applicateur (4).

2. Applicateur chirurgical selon la revendication 1, **caractérisé en ce qu'**à l'état d'insertion, la cartouche d'agrafes (11) complète entièrement la tige tubulaire (2) dans la région de la percée (9).

3. Applicateur chirurgical selon l'une ou l'autre des revendications précédentes, **caractérisé en ce que** la percée (9) s'étend sur une région périphérique de la tige tubulaire (2) allant jusqu'à 180°.

4. Applicateur chirurgical selon l'une des revendications précédentes, **caractérisé en ce que** la percée (9) se raccorde directement à l'outil applicateur (4).

5. Applicateur chirurgical selon l'une des revendications précédentes, **caractérisé en ce que** la percée (9) ne s'étend que sur une petite partie de la longueur de la tige tubulaire.

6. Applicateur chirurgical selon l'une des revendications précédentes, **caractérisé en ce que** la cartouche d'agrafes (11) peut être reliée de manière détachable à la tige tubulaire (2) par une liaison par encliquetage (13, 15, 16) élastique.

7. Applicateur chirurgical selon l'une des revendications précédentes, **caractérisé en ce que** la cartouche d'agrafe (11) porte sur son extrémité tournée vers la poignée (1) une protubérance (12) plongeant dans la tige tubulaire (2).

8. Applicateur chirurgical selon l'une des revendications précédentes, **caractérisé en ce que** le mécanisme d'avancement (34) présente des organes d'entraînement (43, 44) en appui sur les agrafes (35), lesquels peuvent être déplacés conjointement de la distance respective d'une agrafe.

9. Applicateur chirurgical selon la revendication 8, **caractérisé en ce que** tous les organes d'entraînement (43, 44) sont agencés sur un support commun (41).

10. Applicateur chirurgical selon l'une ou l'autre des revendications 8 et 9, **caractérisé en ce que** les organes d'entraînement (43, 44) sont des languettes élastiques qui rebondissent lors du mouvement de retour des organes d'entraînement et glissent le long des agrafes (35) suivantes dans la cartouche d'agrafes (11) sans déplacer celle-ci, et viennent en avançant en appui avec leurs arêtes libres (46) sur la face postérieure d'une agrafe (35).

11. Applicateur chirurgical selon la revendication 10, **caractérisé en ce que** les arêtes (46) sont agencées en biais par rapport au plan des agrafes.

12. Applicateur chirurgical selon l'une ou l'autre des revendications 10 et 11, **caractérisé en ce que** chaque organe d'entraînement présente deux languettes (43, 44) agencées l'une à côté de l'autre.

13. Applicateur chirurgical selon la revendication 12, **caractérisé en ce qu'**entre les deux languettes (43, 44) est agencée une autre languette (45) élastique qui prend appui sur la face inférieure de l'agrafe (35).

14. Applicateur chirurgical selon la revendication 13, **caractérisé en ce que** les deux languettes (43, 44) extérieures et la languette médiane (45) font partie d'une languette (42) plus longue précontrainte élastiquement contre les agrafes (35).

15. Applicateur chirurgical selon l'une quelconque des revendications 9 à 14, **caractérisé en ce que** le support (41) porte deux protubérances (54) orientées vers le bas, entre lesquelles s'engage un organe d'entraînement (51) actionnable par la poignée (1) et déplaçant le support (41).

16. Applicateur chirurgical selon la revendication 15, **caractérisé en ce que** les protubérances sont deux languettes (54) agencées en sens inverse et **en ce que** l'organe d'entraînement s'engage entre leurs arêtes (55) libres.

17. Applicateur chirurgical selon l'une quelconque des revendications 9 à 16, **caractérisé en ce que** la cartouche d'agrafes (11) présente une chambre de réception (36) ouverte vers l'intérieur de la tige tubulaire (2), dans laquelle plusieurs agrafes (35) sont reçues à déplacement les unes derrière les autres le long de la chambre de réception (36), et **en ce que** le support (41) est monté à déplacement longitudinal sur la cartouche d'agrafes (11) en recouvrant la chambre de réception (36) vers la tige tubulaire (2).

18. Applicateur chirurgical selon l'une quelconque des revendications 15 à 17, **caractérisé en ce que** l'organe d'entraînement (51) est relié à un élément d'actionnement (49) déplaçable au moyen d'un ressort (56) jusque dans la position terminale proche de la poignée.

19. Applicateur chirurgical selon l'une quelconque des revendications 9 à 14 ou 17, **caractérisé en ce que** le support (41) est déplacé par un ressort (64) jusque dans une position terminale rétractée et **en ce que** sur le support attaque un élément d'actionnement (49) déplaçant celui-ci à l'encontre de l'effet du ressort (64).

20. Applicateur chirurgical selon la revendication 19, **caractérisé en ce que** le support (41) porte un prolongement (71) qui actionne dans sa position rétractée proche de la poignée un verrou (67) qui ne libère le mécanisme d'avancement (34) qu'à l'état actionné, mais qui autrement le bloque.

21. Applicateur chirurgical selon la revendication 20, **caractérisé en ce que** le verrou (67) est un levier basculant qui, à l'état non actionné, prend appui sur une protubérance (57) du mécanisme d'avancement (34) en le bloquant.

22. Applicateur chirurgical selon l'une quelconque des revendications 18 à 21, **caractérisé en ce que** l'élément d'actionnement est une tige de poussée et de traction (49) agencée dans la tige tubulaire (2).

23. Applicateur chirurgical selon la revendication 22, **caractérisé en ce que** sur la poignée (1) est monté un levier basculant (50) susceptible d'être relié de manière articulée avec la tige de poussée et de traction (49).

24. Applicateur chirurgical selon l'une ou l'autre des revendications 22 et 23, **caractérisé en ce qu'**a la tige de poussée et de traction (49) est associée une course libre (59, 60, 61, 62) qui respectivement bloque la tige de poussée et de traction (49) dans une direction et la libère dans l'autre direction, et **en ce que** la direction de libération n'est inversable que dans une des positions terminales respectives de la tige de poussée et de traction (49).

25. Applicateur chirurgical selon la revendication 24, **caractérisé en ce que** la course libre est formée par un levier de blocage (62) monté à basculement sur la poignée (1) et prenant appui en oblique sur un profilage (59) de la surface périphérique de la tige de poussée et de traction (49), ledit levier de blocage plongeant dans des creux (60, 61) de la tige de poussée et de traction (49) à l'extrémité du profilage (59) perpendiculairement à la surface périphérique et, dans cette position, il est basculé dans une position oblique symétrique orientée à l'inverse lorsque la tige de poussée et de traction (49) est déplacée en sens inverse.

26. Applicateur chirurgical selon l'une quelconque des revendications précédentes, **caractérisé en ce que** la tige tubulaire (2) est amovible dans la partie (10) opposée à la percée (9).

27. Applicateur chirurgical selon la revendication 26, **caractérisé en ce que** la partie antérieure (10) amovible de la tige tubulaire (2) est reliée à son autre partie (17, 21) via une liaison à pince de serrage (18, 19, 20).

28. Applicateur chirurgical selon l'une quelconque des revendications précédentes, **caractérisé en ce que** la tige tubulaire (2) est reliée à la poignée (1) de manière rotative autour de l'axe longitudinal de la tige tubulaire et de manière amovible.

29. Applicateur chirurgical selon la revendication 28, **caractérisé en ce que** la liaison entre la tige tubulaire (2) et la poignée (1) a lieu via un encliquetage à bille (22, 23, 24, 25).

30. Applicateur chirurgical selon l'une quelconque des revendications précédentes, **caractérisé en ce que** le mécanisme de fermeture (8) de l'outil applicateur (4) présente des moyens de transmission reliés à deux mâchoires de pince montées pivotantes qui sont entraînées par une tige de poussée et de traction agencée dans la tige tubulaire (2).

31. Applicateur chirurgical selon la revendication 30, **caractérisé en ce que** la tige de poussée et de traction du mécanisme de fermeture (8) est réalisée sous forme de douille (7) entourant coaxialement la tige de poussée et de traction (49) du mécanisme d'avancement (34).

32. Applicateur chirurgical selon la revendication 31, **caractérisé en ce que** deux bras (5, 6) pivotant l'un à l'encontre de l'autre sont agencés sur la poignée (1) pour déplacer la douille (7).

33. Applicateur chirurgical selon l'une ou l'autre des revendications 31 et 32, **caractérisé en ce que** la douille (7) est découpée dans la région de la percée (9).
